# EUROPEAN PATENT APPLICATION

(11) **EP 2 775 290 A1**
(43) Date of publication of application: **10.09.2014**
(21) Application number: 14156550.7
(22) Date of filing: 25.02.2014
(51) Int. Cl.: G01N 21/64

(54) **Microorganism detecting apparatus and microorganism detecting method**

(30) Priority: 05.03.2013 JP 2013042906
(71) Applicant: Azbil Corporation, Chiyoda-ku Tokyo 100-6419 (JP)
(72) Inventor: Hasegawa, Norio, Tokyo 100-6419 (JP)
(74) Representative: Tischner, Oliver

(57) **Abstract**

The present invention relates to a microorganism detecting device includes a light source (25) that illuminates a particle with an excitation beam; a fluorescence detector (27) that detects fluorescent light emitted from a particle; and a evaluating portion (301) adapted to evaluate a particle as a microorganism if there are multiple peaks in the spectrum of the fluorescent light that has been detected by the fluorescence detector (27), and adapted to evaluate the particle as a non-fluorescent particle if there is a single peak in the spectrum of the fluorescent light.

## Description

The present invention relates to a technology for evaluating an environment, and, in particular, relates to a microorganism detecting device and a microorganism detecting method.

In clean rooms, such as bio clean rooms, airborne microorganisms are detected and recorded using microorganism detecting devices (referencing, for example, JP 2011-83214and N. Hasegawa, et al., "Real-time Detecting Technologies for Airborne Microbes, and Applications Thereof," Yamatake Corporation, azbil Technical Review, December 2009, Pages 2-7, 2009).The state of wear of the air-conditioning equipment of the clean room can be ascertained from the result of the microorganism detection. Moreover, a record of microorganism detection within the clean room may be added as reference documentation to the products manufactured within the clean room. Optical microorganism detecting devices draw in air from a clean room, for example, and illuminate the drawn-in air with light. When there is a microorganism included within the air, a microorganism that is illuminated with light emits fluorescence, enabling detection of the numbers, sizes, and the like, of microorganisms included in the air.

However, it was discovered by the present inventor that there are times wherein, if non-microorganism particles that emit fluorescence are included in the gas, the microorganism detecting device will mistakenly detect the non-microorganism particle as a microorganism. Given this, one object of the present invention is to provide a microorganism detecting device and a microorganism detecting method able to discriminate between microorganism particles and non-microorganism particles.

An aspect of the present invention provides a microorganism detecting device comprising: (a) a light source for illuminating a particle with an excitation beam; (b) a fluorescence detector for detecting fluorescent light emitted from a particle; and (c) an evaluating portion for identifying a particle as a microorganism if there are multiple peaks in the spectrum of the florescent light, and for identifying the particle as a non-fluorescent particle if there is a single peak in the spectrum of the florescent light. Note that "fluorescent light" includes a autofluorescent light.

Moreover, an aspect of the present invention provides a microorganism detecting method including: (a) illuminating a particle with an excitation beam; (b) detecting fluorescence emitted by the particle, and (c) identifying a particle as a microorganism if there are multiple peaks in the spectrum of the florescent light, and for identifying the particle as a non-fluorescent particle if there is a single peak in the spectrum of the florescent light. According, to an embodiment a microorganism detecting device is provided comprising: a light source for illuminating a particle with an excitation beam; a fluorescence detector for detecting fluorescent light emitted from a particle; and a evaluating portion for identifying a particle as a microorganism if there are multiple peaks in the spectrum of the florescent light that has been detected by the fluorescence detector, and for identifying the particle as a non-fluorescent particle if there is a single peak in the spectrum of the florescent light.

The present invention enables provision of a microorganism detecting device and a microorganism detecting method able to discriminate between microorganism particles and non-microorganism particles.

Further advantages, features, aspects and details are evident from the dependent claims, the description and the drawings.

### Brief Descriptions of the Drawings

So that the manner in which the above recited features of the present invention can be understood in detail, a more particular description of the invention, briefly summarized above, may be read by reference to embodiments. The accompanying drawings relate to embodiments of the invention and are described in the following:
FIG. 1 is a schematic diagram of a clean room according to a form of embodiment according to the present invention.
FIG. 2 is a schematic cross-sectional diagram of a microorganism detecting device according to a form of embodiment according to the present invention.
FIG. 3 is an excitation emission matrix for *Escherichia coli* according to an embodiment according to the present invention.
FIG. 4 is an excitation emission matrix for polyester clean room wear according to an embodiment according to the present invention.
FIG. 5 is a fluorescence spectrum for *Escherichia coli* according to an embodiment according to the present invention.
FIG. 6 is a first derivative of a fluorescence spectrum for *Escherichia coli* according to an embodiment according to the present invention.
FIG. 7 is a second derivative of a fluorescence spectrum for *Escherichia coli* according to an embodiment according to the present invention.
FIG. 8 is a third derivative of a fluorescence spectrum for *Escherichia coli* according to an embodiment according to the present invention.
FIG. 9 is a fluorescence spectrum for the polyester clean room wear according to an embodiment according to the present invention.
FIG. 10 is a first derivative of a fluorescence spectrum for the polyester clean room wear according to an embodiment according to the present invention.
FIG. 11 is a second derivative of a fluorescence spectrum for the polyester clean room wear according to an embodiment according to the present invention.
FIG. 12 is a third derivative of a fluorescence spectrum for the polyester clean room wear according to an embodiment according to the present invention.
FIG. 13 is a second derivative of a fluorescence spectrum for *Escherichia coli* according to an embodiment according to the present invention.
FIG. 14 is a second derivative of a fluorescence spectrum for the polyester clean room wear according to an embodiment according to the present invention.
FIG. 15 is a table of values wherein, in the derivative spectrum where, in the second derivative of the fluorescent light spectrum in an embodiment according to the present invention, where the absolute value of the derivative value of the florescent light intensity at a peak that could derive from the fluorescent light that is emitted from a microorganism is divided by the absolute value of the derivative of a magnitude of a fluorescent intensity that is not significant.
FIG. 16 is a third derivative of a fluorescence spectrum for *Escherichia coli* according to an embodiment according to the present invention.
FIG. 17 is a third derivative of a fluorescence spectrum for the polyester clean room wear according to an embodiment according to the present invention.
FIG. 18 is a table of values wherein, in the derivative spectrum where, in the third derivative of the fluorescent light spectrum in an embodiment according to the present invention, where the absolute value of the derivative value of the florescent light intensity at a peak that could derive from the fluorescent light that is emitted from a microorganism is divided by the absolute value of the derivative of a magnitude of a fluorescent intensity that is not significant.
FIG. 19 is a second derivative of a fluorescence spectrum for *Escherichia coli* according to an embodiment according to the present invention.
FIG. 20 is a second derivative of a fluorescence spectrum for the polyester clean room wear according to an embodiment according to the present invention.
FIG. 21 is a table of values wherein, in the derivative spectrum where, in the second derivative of the fluorescent light spectrum in an embodiment according to the present invention, where the area of a peak that could derive from the fluorescent light that is emitted from a microorganism is divided by the area of a peak of a fluorescent intensity that is not significant.
FIG. 22 is a second derivative of a fluorescence spectrum for *Escherichia coli* according to an embodiment according to the present invention.
FIG. 23 is a second derivative of a fluorescence spectrum for the polyester clean room wear according to an embodiment according to the present invention.
FIG. 24 is a table of values wherein, in the derivative spectrum where, in the second derivative of the fluorescent light spectrum in an embodiment according to the present invention, where the area of a peak that could derive from the fluorescent light that is emitted from a microorganism is divided by the area of a peak of a fluorescent intensity that is not significant.
FIG. 25 is a third derivative of a fluorescence spectrum for *Escherichia coli* according to an embodiment according to the present invention.
FIG. 26 is a third derivative of a fluorescence spectrum for the polyester clean room wear according to an embodiment according to the present invention.
FIG. 27 is a table of values wherein, in the derivative spectrum where, in the third derivative of the fluorescent light spectrum in an embodiment according to the present invention, where the area of a peak that could derive from the fluorescent light that is emitted from a microorganism is divided by the area of a peak of a fluorescent intensity that is not significant.
FIG. 28 is a third derivative of a fluorescence spectrum for *Escherichia coli* according to an embodiment according to the present invention.
FIG. 29 is a third derivative of a fluorescence spectrum for the polyester clean room wear according to an embodiment according to the present invention.
FIG. 30 is a table of values wherein, in the derivative spectrum where, in the third derivative of the fluorescent light spectrum in an embodiment according to the present invention, where the area of a peak that could derive from the fluorescent light that is
emitted from a microorganism is divided by the area of a peak of a fluorescent intensity that is not significant. Embodiments of the Present Invention

A form of embodiment of the present invention will be described below. In the descriptions of the drawings below, identical or similar components are indicated by identical or similar codes. Note that the diagrams are schematic. Consequently, specific measurements should be evaluated in light of the descriptions below. Furthermore, even within these drawings there may, of course, be portions having differing dimensional relationships and proportions.

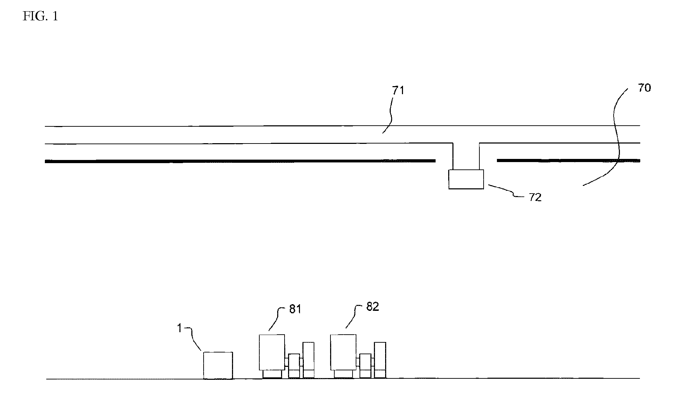

As illustrated in FIG. 1, a microorganism detecting device 1 according to the present embodiment is disposed in, for example, a clean room 70. In the clean room 70, clean air is blown in through a duct 71 and a through blowing opening 72 having an ultrahigh performance air filter such as a HEPA filter (High Efficiency Particulate Air Filter) or ULPA filter (Ultra Low Penetration Air Filter), or the like.

Manufacturing lines 81 and 82 are arranged inside of the clean room 70. The manufacturing lines 81 and 82 are a manufacturing lines, for, for example, precision instruments, electronic components, or semiconductor devices. Conversely, the manufacturing lines 81 and 82 may be manufacturing lines for foodstuffs, beverages, or pharmaceuticals. For example, in the manufacturing lines 81 and 82, an infusion liquid may be filled into an intravenous infusion device or a hypodermic. Conversely, the manufacturing lines 81 and 82 may manufacture oral medications or Chinese herb medications. On the other hand, the manufacturing lines 81 and 82 may fill containers with a vitamin drink or beer.

The manufacturing lines 81 and 82 normally are controlled so that microorganisms and non-microorganism particles, and the like, are not dispersed into the air within the clean room 70. However, manufacturing lines 81 and 82, for some reason, are sources that produce microorganisms and non-microorganism particles that become airborne in the clean room 70. Moreover, factors other than the manufacturing lines 81 and 82 also disperse microorganisms and non-microorganism particles into the air of the clean room 70.

Examples of microorganisms that may become airborne in the clean room 70 include microbes. Examples of such microbes include Gram-negative bacteria, Gram-positive bacteria, and fungi such as mold spores. *Escherichia coli,* for example, can be listed as an example of a Gram-negative bacterium. *Staphylococcus epidermidis, Bacillus atrophaeus, Micrococcus lylae,* and *Corynebacterium afermentans* can be listed as examples of Gram-positive bacteria. *Aspergillus niger* can be listed as an example of a fungus such as a mold spore. However, the microorganisms that may become airborne in the clean room 70 are not limited to these specific examples. Examples of non-microorganism particles that may become airborne in the clean room 70 include splashed chemical substances, pharmaceuticals, or foodstuffs, along with dust, dirt, grime, and the like.

If a microorganism is illuminated with light, the nicotinamide adenine dinucleotide and the riboflavin, and the like, that are included in microorganisms produce fluorescent light. Conventionally, devices that detect microorganisms in a gas would detect as microorganisms particles in the air that, when illuminated with light, emit fluorescence. However, there are cases wherein fluorescent particles that fall off of a gown, made from a polyester material, for example, that has been cleaned will emit fluorescence when illuminated with light, even though they are non-microorganism particles. Moreover, polystyrene particles also emit fluorescence, and then fade. Because of this, in the conventional devices for detecting airborne microorganisms, there are times wherein non-microorganism particles that are airborne and that emit fluorescence are detected in error as microorganisms.

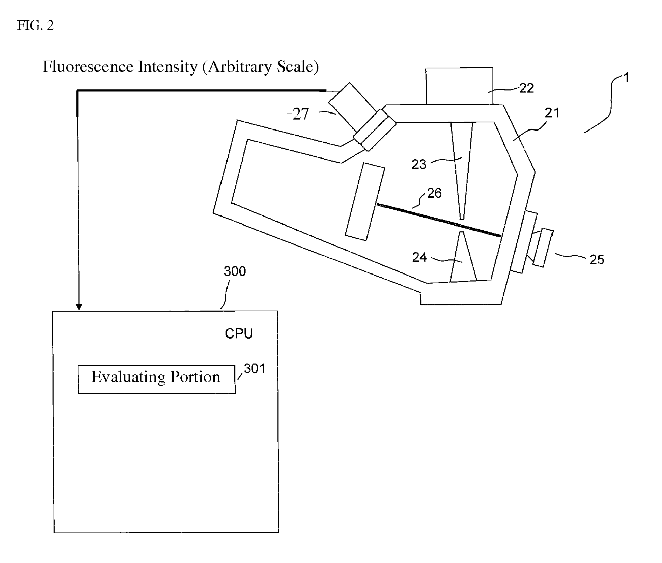

In contrast, as illustrated in FIG. 2, the microorganism detecting device 1 according to the present embodiment includes: a light source 25 for illuminating a particle with an excitation beam; a fluorescence detector 27 for detecting fluorescent light emitted from a particle; and an evaluating portion 301 for identifying a particle as a microorganism if there are multiple peaks in the spectrum of the florescent light that has been detected by the fluorescence detector 27, and for identifying the particle as a non-fluorescent particle if there is a single peak in the spectrum of the florescent light that has been detected by the fluorescence detector 27.

The light source 25 and the fluorescence detector 27 are provided in the frame 21. The microorganism detecting device 1 is further provided with a first vacuum device 22 for drawing air from within the clean room 70 into the interior of the frame 21.The air drawn in by the first vacuum device 22 is expelled from the tip of a nozzle 23 of a flow path within the frame 21. The air that is expelled from the tip of the nozzle 23 is drawn in by a second vacuum device 24, disposed within the frame 21, facing the tip of the nozzle 23.

The light source 25 directs a laser beam 26 of a broad wavelength band toward the air that is that is expelled from the tip of the nozzle 23 and that is drawn by the second vacuum device 24.The wavelengths of the laser beam 26 are, for example, from 250 to 550 nm. The laser beam 26 may be of visible light, or may be of ultraviolet radiation. If the laser beam 26 is of visible light, then the wavelength of the laser beam 26 is within a range of, for example, between 400 and 550 nm, for example, 405 nm. If the laser beam 26 is of ultraviolet radiation, then the wavelength of the laser beam 26 is in a range of, for example, between 300 and 380 nm, for example, 340 nm. However, the wavelengths of the laser beam 26 are not limited thereto.

When microorganisms, such as microbes, or the like, exist within the air that is blown out from the nozzle 23, then the microbes that are exposed to the laser beam 26 produce fluorescence. Moreover, when non-microorganism particles, such as polyester particles, or the like, also exist within the air that is blown out from the nozzle 23, then the non-microorganism particles that are exposed to the laser beam 26 produce fluorescence. The fluorescence detector 27 detects the fluorescence produced by the microorganisms and the non-microorganism particles.

Microorganisms include fluorescent substances such as riboflavin, flavin nucleotides (FMN), flavin adenine dinucleotide (FAD), nicotinamide adenine dinucleotide phosphate (NAD(P)H), pyridoxamine, pyridoxal phosphate (pyridoxal-5 '-phosphate), pyridoxine, tryptophan, tyrosine, phenylalanine, and the like.

The riboflavin, flavin nucleotides, and flavin adenine dinucleotides emit fluorescence at between 510 and 540 nm. The nicotinamide adenine dinucleotide phosphate emits fluorescence at between 440 and 480 nm. Depending on the excitation beam, the pyridoxamine, pyridoxal phosphate, and pyridoxine emit fluorescence at between 380 and 410 nm or between 480 and 520 nm. The tryptophan emits fluorescence at between 330 and 360 nm. The tyrosine emits fluorescence at between 290 and 320 nm. The phenylalanine emits fluorescence at between 270 and 300 nm.

Consequently, when the particles that are included in the air that is blown out from the nozzle 23 are microorganisms, the fluorescent spectra produced by the microorganisms that are illuminated with the laser beam 26 with the broad wavelength band will have multiple peaks. In contrast, the florescent non-microorganism particles produced by the clean wear of the operators within the clean room 70 are usually made from only one type of fluorescent material. Because of this, when the particles that are included in the air that is blown out from the nozzle 23 are non-microorganism particles, the fluorescence spectrum produced by the non-microorganism particles that are illuminated with the laser beam 26 with the broad wavelength band will have only a single peak.

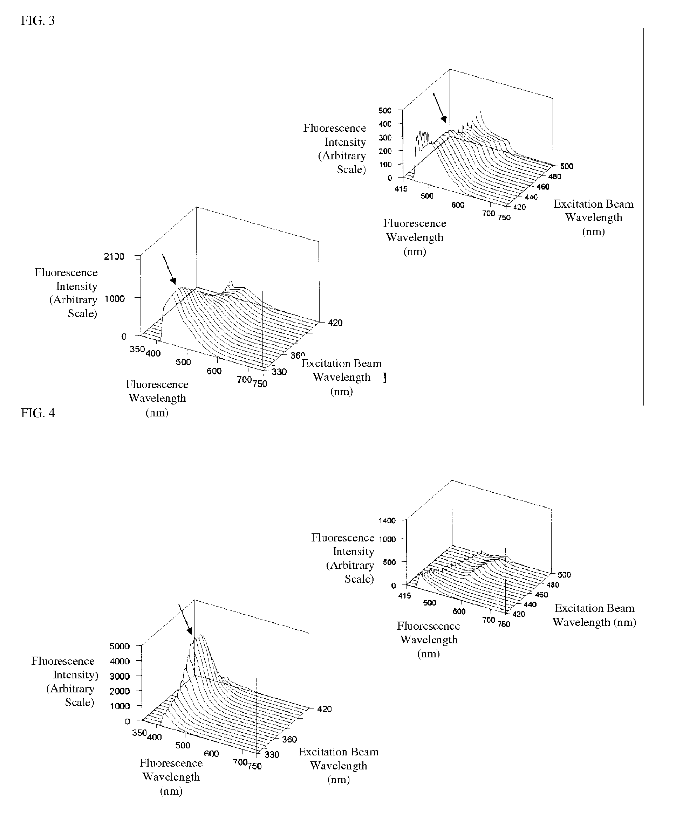

FIG. 3 is one embodiment of an excitation emission matrix for E. *coli,* and FIG. 4 is one embodiment of an excitation emission matrix for clean room wear made out of a polyester material. In the excitation emission matrix, the x axis represents the excitation beam wavelength, the y axis represents the fluorescence wavelength, and the z axis represents the fluorescence intensity, where the fluorescence spectrum is included. In FIG. 3 and FIG. 4, the peaks indicated by the arrows are significant peaks from fluorescence, and the other peaks are peaks due to noise, from stray light, scattered light, or the like. As illustrated in FIG. 3, there are at least two significant peaks in the fluorescence spectrum for E. *coli,* but in FIG. 4 there is only a single significant peak in the fluorescent spectrum for the polyester clean room wear.

The evaluating portion 301 may be, for example, included in a central calculation processing device (CPU) 300.The evaluating portion 301 receives an instantaneous fluorescence spectrum from, for example, a fluorescence detector 27, and evaluates whether or not there are multiple peaks in the fluorescence spectrum. If there is a plurality of peaks in the fluorescence spectrum, then the evaluating portion 301 evaluates that the particle is a microorganism. If there is a single peak in the fluorescence spectrum, then the evaluation is that the particle is a non-microorganism particle. Note that the evaluating portion 301 excludes in advance, from consideration in the evaluation, electrical noise included in the fluorescence spectrum that could derive from the electrical circuitry of, for example, the fluorescence detector 27. The method for excluding the noise may use, for example, the least-squares method, the simple moving average method, the Savitzky-Golay method, or the like.

Furthermore, the evaluating portion 301 may determine whether or not there is a plurality of peaks included in the fluorescence spectrum by taking the derivative of the fluorescence spectrum. For example, in the odd-number derivative spectra, such as the first, third, etc., the derivative value for the fluorescent intensity at the peak wavelength of the original fluorescence spectrum will go to zero. Consequently, the evaluating portion 301 may evaluate whether or not there are multiple peaks included in the original fluorescence spectrum by counting the wavelengths wherein the derivative value of the fluorescence intensity, in the derivative spectrum, goes to zero.

Moreover, peaks that are included in the original fluorescence spectrum will be more clearly visible in a derivative spectrum of an even-number derivative, such as the second or fourth derivative, wherein the derivative of the fluorescence spectrum has been taken an even number of times. Consequently, the evaluating portion 301 may evaluate whether or not there is a plurality of peaks included in the original fluorescence spectrum through counting the number of peaks in an even-number derivative spectrum.

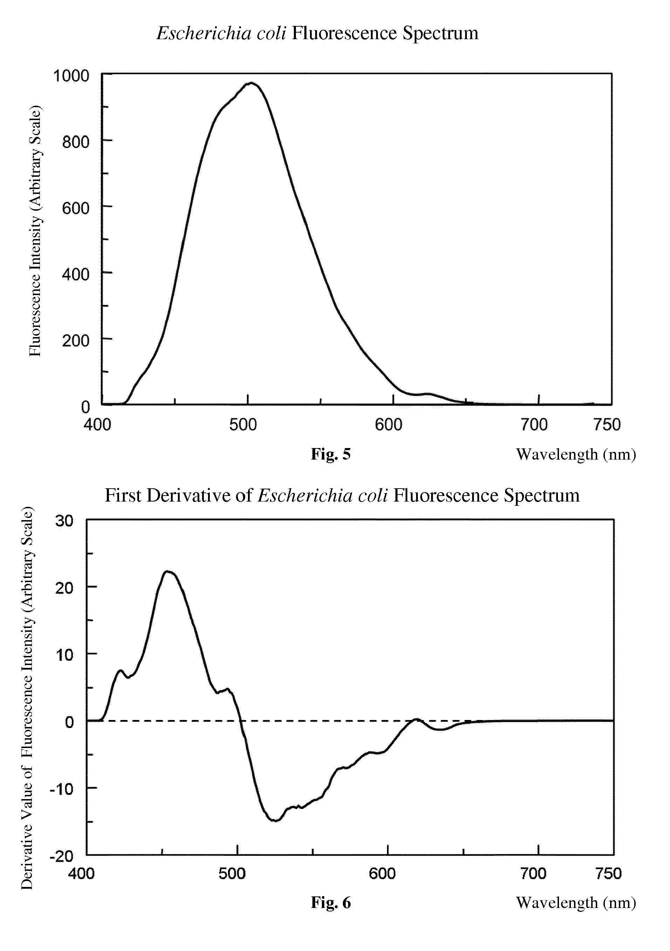

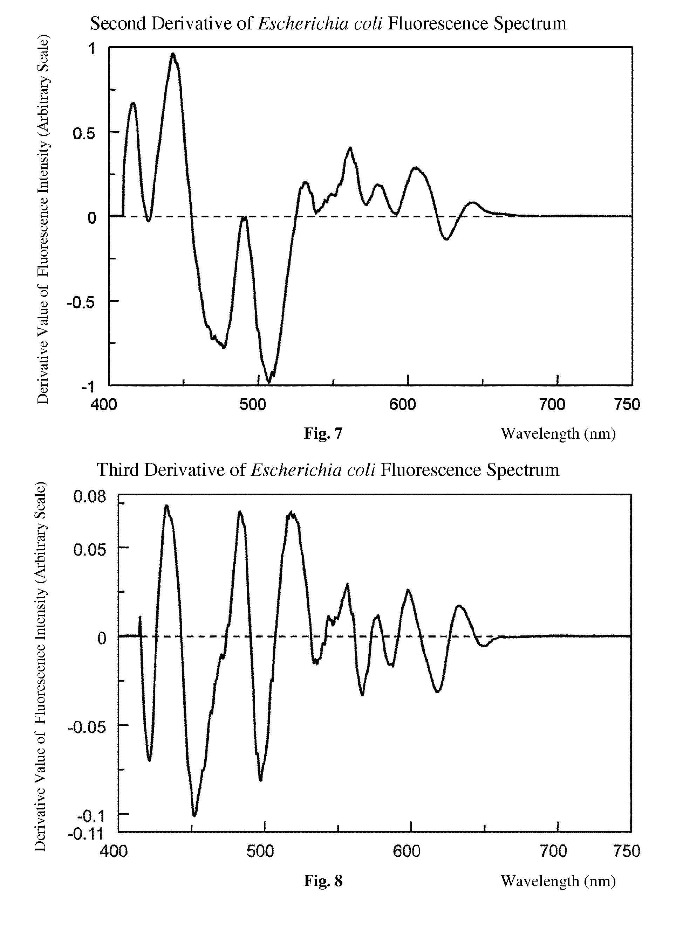

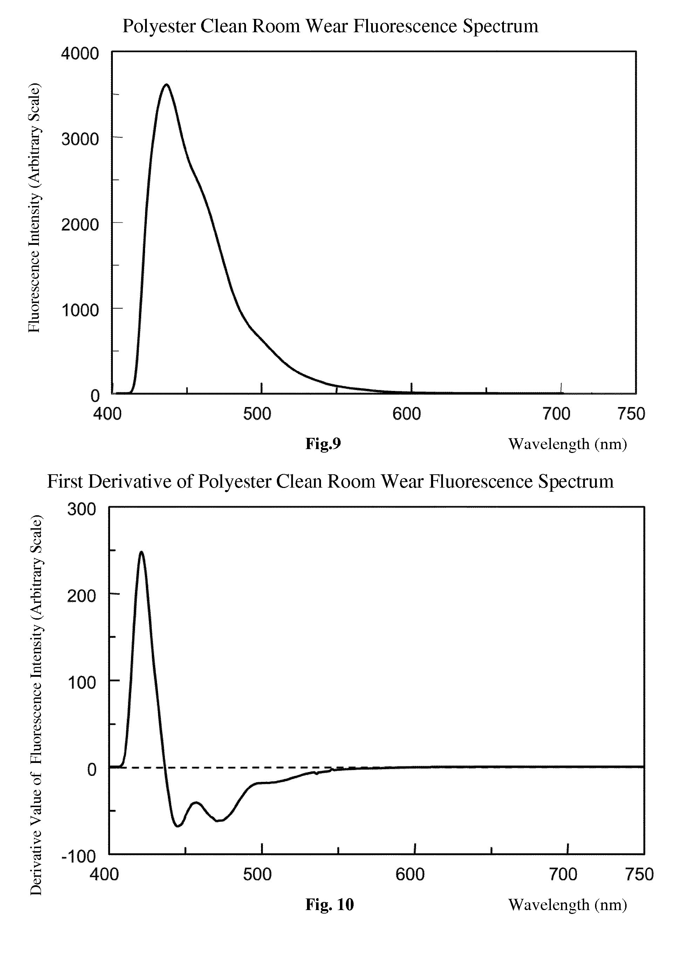

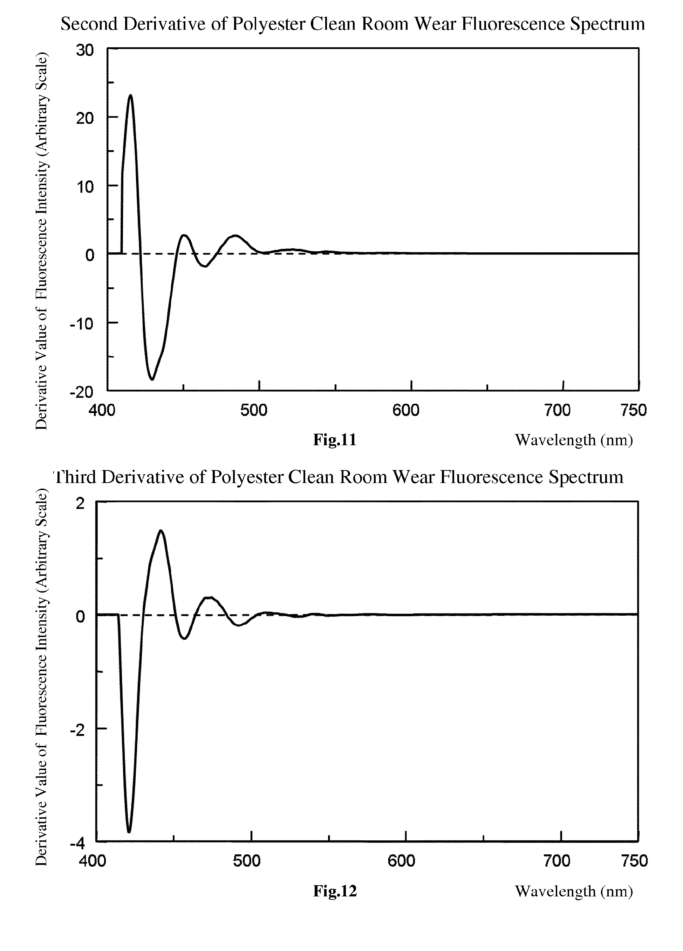

FIG. 5 is an embodiment for the fluorescence spectrum of *E*. *coli*, FIG. 6 is an embodiment for the first derivative of the fluorescence spectrum of *E*. *coli,* FIG. 7 is an embodiment for the second derivative of the fluorescence spectrum of *E*. *coli,* and FIG. 8 is an embodiment for the third derivative of the fluorescence spectrum of *E*. *coli.* Moreover, FIG. 9 is an embodiment for the fluorescence spectrum of a polyester clean room wear, FIG. 10 is an embodiment for the first derivative of the fluorescence spectrum of the polyester clean room wear, FIG. 11 is an embodiment for the second derivative of the fluorescence spectrum of the polyester clean room wear, and FIG. 12 is an embodiment for the third derivative of the fluorescence spectrum of the polyester clean room wear.

Taking multiple derivatives of a fluorescence spectrum tends to reduce the variability of the peak heights in the original spectrum, making it easier to detect the number of peaks. Moreover, taking multiple derivatives of the fluorescence spectrum also makes it possible to isolate the peaks that are not separated, known as "shoulders," that are touching each other in the original spectrum.

Furthermore, the evaluating portion 301 may compare, to a discriminant value (a threshold value) that is set in advance, the absolute value P_{E} of the derivative value of the fluorescent intensity of a peak that could derive from fluorescence emitted from a microorganism in the derivative spectrum where the derivative of the fluorescence spectrum has been taken, and may evaluate a particle as a microorganism if the absolute value P_{E} of the derivative value of the fluorescent intensity is larger than the discriminant value, and evaluate the particle to be a non-microorganism particle if the absolute value P_{E} of the derivative value of the fluorescent intensity is smaller than the discriminant value. Note that the evaluating portion 301 may compare, to a discriminant value that is set in advance, a value for P_{E}/P_{N} wherein the absolute value P_{E} of the derivative value of the fluorescent intensity at a peak that could derive from fluorescence emitted by a microorganism is divided by the absolute value P_{N} of the derivative value of the magnitude of the fluorescent intensity that is not significant in the derivative spectrum. Doing so makes it possible to suppress errors deriving from fluorescent intensity that is not significant.

Note that here the fluorescent intensity that is not significant indicates the fluorescent intensity at peaks that appear at wavelengths that are other than the peak wavelengths of the fluorescence that could be produced by the fluorescent substances included in microorganisms, such as the fluorescent intensity in a peak due to noise or the fluorescent intensity of the peak that appears at the cutoff wavelength of a long-pass filter. Moreover, a peak that could derive from the fluorescent intensity emitted by a microorganism is, for example, a peak at a wavelength of fluorescence that is emitted from a microorganism when the derivative spectrum is an even-number derivative spectrum. Moreover, when the derivative spectrum is an odd-number derivative spectrum it is a peak that is adjacent to the wavelength of the fluorescence that is emitted from the microorganism.

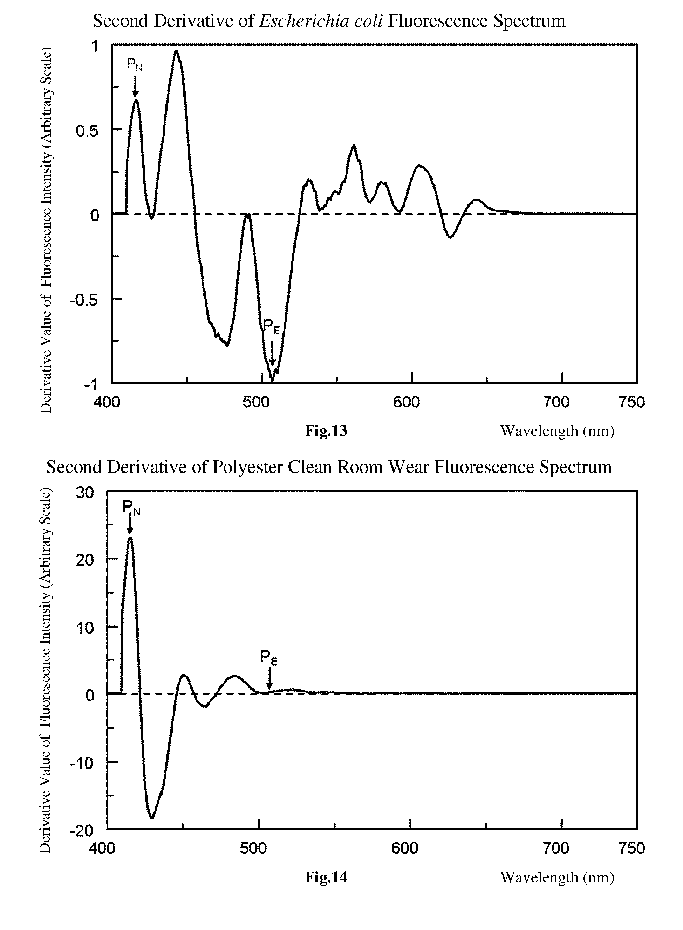

In the embodiment of a second derivative of the fluorescence peak for *E*. *coli,* shown in FIG. 13, and in the embodiment of a second derivative of the fluorescence spectrum of the polyester clean room wear shown in FIG. 14, the peak P_{N} that appears in the vicinity of a wavelength of 420 nm is a peak that is not significant, appearing due to the light of less than the 420 nm wavelength that is blocked by the long-pass filter. Moreover, in the embodiment of a second derivative of the fluorescence peak for *E*. *coli*, shown in FIG. 13, the peak P_{E} that appears near the wavelength of 510 nm is a peak that derives from fluorescent light that is emitted from a microorganism, resulting from the riboflavin. If it is not clear whether a fluorescence spectrum is due to a microorganism or due to a non-microorganism particle, the peak P_{E} that appears here the wavelength of 510 nm, in the embodiment of a second derivative of the fluorescence spectrum of the polyester clean room wear shown in FIG. 14 is a peak that could derive from the fluorescence emitted from a microorganism as the result of the riboflavin.

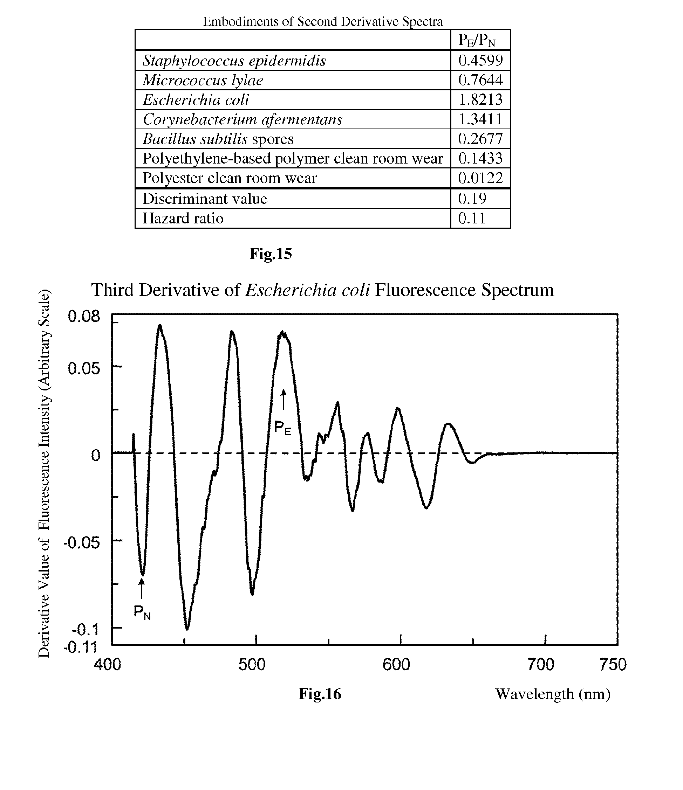

As shown in FIG. 15, the value for P_{E/}P_{N}, wherein the absolute value of the derivative value of the fluorescent intensity at the peak P_{E} that appears near to the wavelength of 510 nm in the embodiment of the second derivative of the fluorescence spectrum for E. *coli,* illustrated in FIG. 13, was divided by the fluorescence intensity that is not significant at the peak P_{N} that appears near to the wavelength of 420 nm was 1.8213. In an embodiment of the second derivative of the fluorescence spectrum for *Staphylococcus epidermidis*, prepared separately, the value of P_{E}/P_{N} was 0.4599. In an embodiment of the second derivative of the fluorescence spectrum for *Micrococcus lylae,* prepared separately, the value of P_{E}/P_{N} was 0.7644. In an embodiment of the second derivative of the fluorescence spectrum for *Corynebacterium afermentans,* prepared separately, the value of P_{E}/P_{N} was 1.3411. In an embodiment of the second derivative of the fluorescence spectrum for *Bacillus subtilis* spores, prepared separately, the value of P_{E}/P_{N} was 0.2677.

In contrast, as illustrated in FIG. 15, the value for P_{E}/P_{N} in an embodiment of a second derivative of the fluorescence spectrum for the polyester clean room wear illustrated in FIG. 14 was 0.0112. In an embodiment of the second derivative of the fluorescence spectrum for the polyethylene-based polymer clean room wear, prepared separately, the value of P_{E}/P_{N} was 0.1433. In this case, when univariate discriminant analysis is performed, the discriminant value was 0.19, and the hazard rate was 0.11. That is, this indicates that if the value of P_{E}/P_{N} is greater than 0.19 the evaluation can be that the particle is a microorganism, with a hazard rate of 0.11.

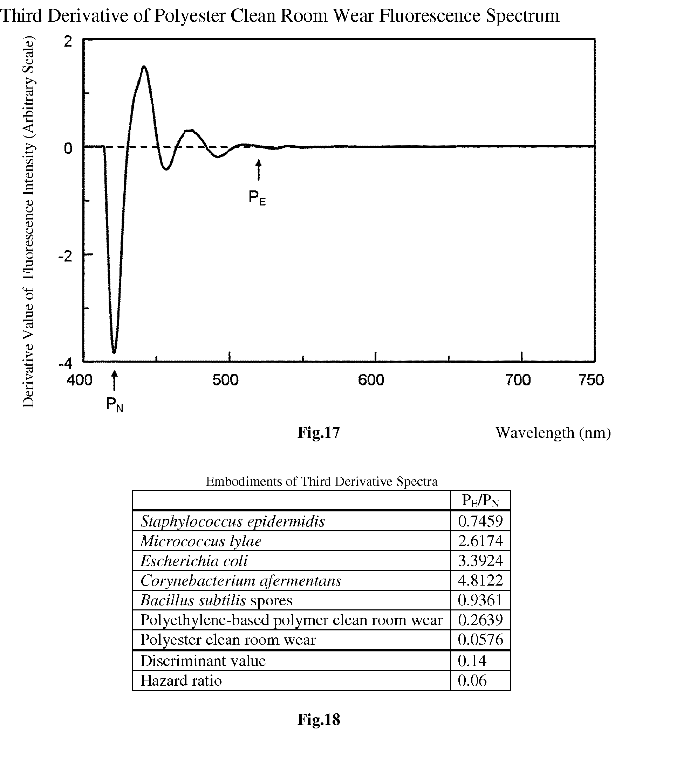

Next, in the embodiment of a third derivative of the fluorescence peak for E. *coli,* shown in FIG. 16, and in the embodiment of a third derivative of the fluorescence spectrum of the polyester clean room wear shown in FIG. 17, the peak P_{N} that appears in the vicinity of a wavelength of 420 nm is a peak that is not significant, appearing due to the light of less than the 420 nm wavelength that is blocked by the long-pass filter. Moreover, in the embodiment of a third derivative of the fluorescence peak for *E*. *coli,* shown in FIG. 16, the peak P_{E} that appears near the wavelength of 510 nm is a peak that derives from fluorescent light that is emitted from a microorganism, resulting from the riboflavin. If it is not clear whether a fluorescence spectrum is due to a microorganism or due to a non-microorganism particle, the peak P_{E} that appears here the wavelength of 510 nm, in the embodiment of a third derivative of the fluorescence spectrum of the polyester clean room wear shown in FIG. 17 is a peak that could derive from the fluorescence emitted from a microorganism as the result of the riboflavin.

As shown in FIG. 18, the value for P_{E}/P_{N}, wherein the absolute value of the derivative value of the fluorescent intensity at the peak P_{E} that appears near to the wavelength of 510 nm in the embodiment of the third derivative of the fluorescence spectrum for *E*. *coli,* illustrated in FIG. 16, was divided by the fluorescence intensity that is not significant at the peak P_{N} that appears near to the wavelength of 420 nm was 3.3924. In an embodiment of the third derivative of the fluorescence spectrum for *Staphylococcus epidermidis,* prepared separately, the value of P_{E}/P_{N} was 0.7459. In an embodiment of the third derivative of the fluorescence spectrum for *Micrococcus lyalae,* prepared separately, the value of P_{E}/P_{N} was 2.6174. In an embodiment of the third derivative of the fluorescence spectrum for *Corynebacterium afermentans,* prepared separately, the value of P_{E}/P_{N} was 4.8122. In an embodiment of the third derivative of the fluorescence spectrum for *Bacillus subtilis* spores, prepared separately, the value of P_{E}/P_{N} was 0.9361.

In contrast, as illustrated in FIG. 18, the value for P_{E}/P_{N} in an embodiment of a third derivative of the fluorescence spectrum for the polyester clean room wear illustrated in FIG. 17 was 0.0576. In an embodiment of the third derivative of the fluorescence spectrum for the polyethylene-based polymer clean room wear, prepared separately, the value of P_{E}/P_{N} was 0.2639. In this case, when univariate discriminant analysis is performed, the discriminant value was 0.14, and the hazard rate was 0.06. That is, this indicates that if the value of P_{E}/P_{N} is greater than 0.14 the evaluation can be that the particle is a microorganism, with a hazard rate of 0.06.

Furthermore, the evaluating portion 301 may compare, to a discriminant value that is set in advance, the area S_{E} of a peak that could derive from fluorescence emitted from a microorganism in the derivative spectrum where the derivative of the fluorescence spectrum has been taken, and may evaluate a particle as a microorganism if the area S_{E} of the peak is larger than the discriminant value, and evaluate the particle to be a non-microorganism particle if the area S_{E} of the peak is smaller than the discriminant value. Note that the evaluating portion 301 may compare, to a discriminant value that is set in advance, a value for S_{E}/S_{N} wherein the area S_{E} of a peak that could derive from fluorescence emitted by a microorganism is divided by the area S_{N} of a peak that is not significant in the derivative spectrum.

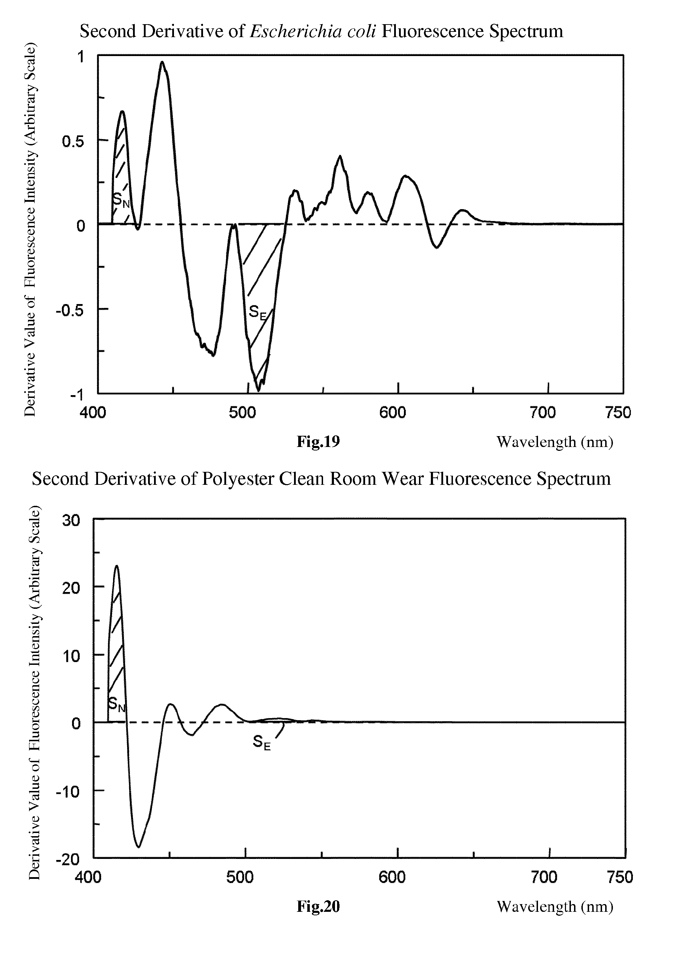

In the embodiment of a second derivative of the fluorescence peak for *E*. *coli,* shown in FIG. 19, and in the embodiment of a second derivative of the fluorescence spectrum of the polyester clean room wear shown in FIG. 20, the peak area S_{N} that appears in the vicinity of a wavelength of 420 nm is an area of a peak that is not significant, appearing due to the light of less than the 420 nm wavelength that is blocked by the long-pass filter when the derivative value 0 of the fluorescence intensity is used as the base. Moreover, in the embodiment of a second derivative of the fluorescence peak for *E*. *coli,* shown in FIG. 19, the peak area S_{E} that appears near the wavelength of 510 nm is an area of a peak that could derive from fluorescent light that is emitted from a microorganism, resulting from the riboflavin when the derivative value 0 of the fluorescence intensity is used as the base. If it is not clear whether a fluorescence spectrum is due to a microorganism or due to a non-microorganism particle, the peak area S_{E} that appears here the wavelength of 510 nm, in the embodiment of a second derivative of the fluorescence spectrum of the polyester clean room wear shown in FIG. 20 is an area of a peak that could derive from the fluorescence emitted from a microorganism as the result of the riboflavin when the derivative value 0 of the fluorescence intensity is used as the base.

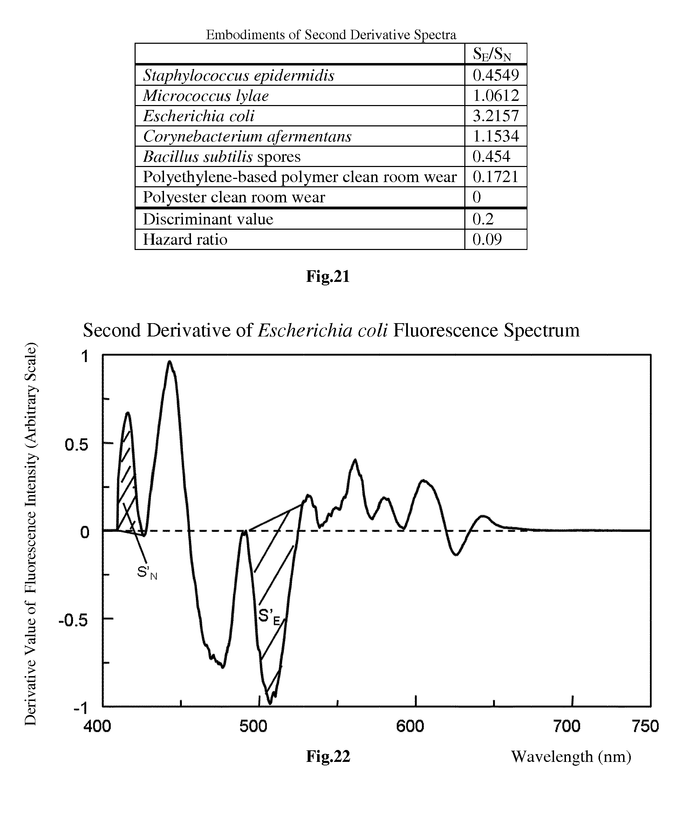

As shown in FIG. 21, the value for S_{E}/S_{N}, wherein the peak area S_{E} that appears near to the wavelength of 510 nm, which could derive from fluorescence emitted by a microorganism due to the riboflavin, in the embodiment of the second derivative of the fluorescence spectrum for *E*. *coli,* illustrated in FIG. 19, was divided by the area of the peak S_{N} that appears near to the wavelength of 420 nm, was 3.2157. In an embodiment of the second derivative of the fluorescence spectrum for *Staphylococcus epidermidis*, prepared separately, the value of S_{E}/S_{N} was 0.4549. In an embodiment of the second derivative of the fluorescence spectrum for *Micrococcus lylae,* prepared separately, the value of S_{E}/S_{N} was 1.0612. In an embodiment of the second derivative of the fluorescence spectrum for *Corynebacterium afermentans,* prepared separately, the value of S_{E}/S_{N} was 1.1534. In an embodiment of the second derivative of the fluorescence spectrum for *Bacillus subtilis* spores, prepared separately, the value of S_{E}/S_{N} was 0.4540.

In contrast, as illustrated in FIG. 21, the value for S_{E}/S_{N} in an embodiment of a second derivative of the fluorescence spectrum for the polyester clean room wear illustrated in FIG. 20 was 0. In an embodiment of the second derivative of the fluorescence spectrum for the polyethylene-based polymer clean room wear, prepared separately, the value of S_{E}/S_{N} was 0.1721. In this case, when univariate discriminant analysis is performed, the discriminant value was 0.20, and the hazard rate was 0.09. That is, this indicates that if the value of S_{E}/S_{N} is greater than 0.20 the evaluation can be that the particle is a microorganism, with a hazard rate of 0.09.

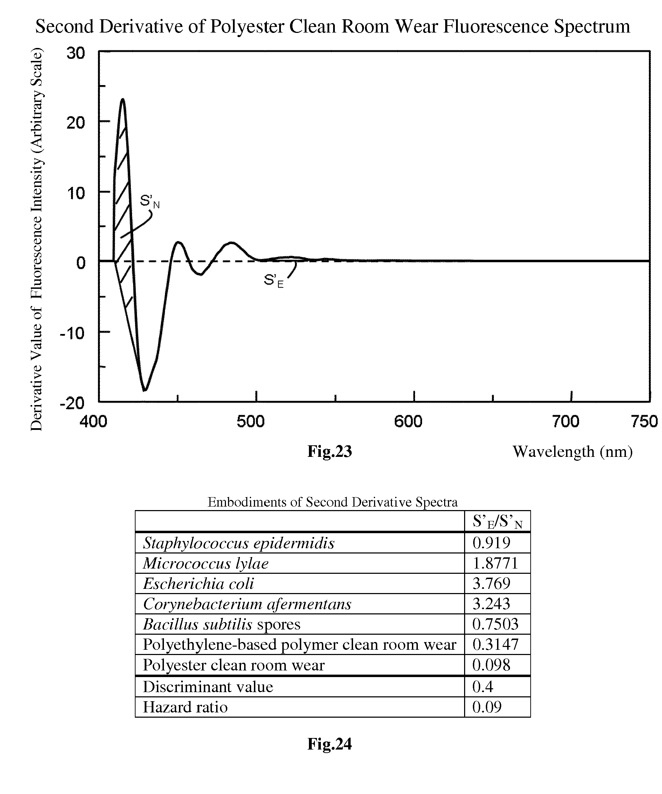

Next, in the embodiment of a second derivative of the fluorescence peak for E. *coli,* shown in FIG. 22, and in the embodiment of a second derivative of the fluorescence spectrum of the polyester clean room wear shown in FIG. 23, the peak area S'_{N} that appears in the vicinity of a wavelength of 420 nm is an area of a peak that is not significant, appearing due to the light of less than the 420 nm wavelength that is blocked by the long-pass filter when a line segment connecting the bottom edges of two peaks is used as the base. Moreover, in the embodiment of a second derivative of the fluorescence peak for E. *coli,* shown in FIG. 22, the peak area S'_{E} that appears near the wavelength of 510 nm is an area of a peak that could derive from fluorescent light that is emitted from a microorganism, resulting from the riboflavin when a line segment connecting the bottom edges of two peaks is used as the base. If it is not clear whether a fluorescence spectrum is due to a microorganism or due to a non-microorganism particle, the peak area S'_{E} that appears here the wavelength of 510 nm, in the embodiment of a second derivative of the fluorescence spectrum of the polyester clean room wear shown in FIG. 23 is an area of a peak that could derive from the fluorescence emitted from a microorganism as the result of the riboflavin when a line segment connecting the bottom edges of two peaks is used as the base.

As shown in FIG. 24, the value for S'_{E}/S'_{N}, wherein the peak area S'_{E} that appears near to the wavelength of 510 nm, which could derive from fluorescence emitted by a microorganism due to the riboflavin, in the embodiment of the second derivative of the fluorescence spectrum for *E*. *coli,* illustrated in FIG. 22, was divided by the area of the peak S'_{N} that appears near to the wavelength of 420 nm, was 3.7690. In an embodiment of the second derivative of the fluorescence spectrum for *Staphylococcus epidermidis*, prepared separately, the value of S'_{E}/S'_{N} was 0.9190. In an embodiment of the second derivative of the fluorescence spectrum for *Micrococcus lylae,* prepared separately, the value of S'_{E}/S'_{N} was 1.8771. In an embodiment of the second derivative of the fluorescence spectrum for *Corynebacterium afermentans,* prepared separately, the value of S'_{E}/S'_{N} was 3.2430. In an embodiment of the second derivative of the fluorescence spectrum for *Bacillus subtilis* spores, prepared separately, the value of S'_{E}/S'_{N} was 0.7503.

In contrast, as illustrated in FIG. 24, the value for S'_{E}/S'_{N} in an embodiment of a second derivative of the fluorescence spectrum for the polyester clean room wear illustrated in FIG. 23 was 0.0980. In an embodiment of the second derivative of the fluorescence spectrum for the polyethylene-based polymer clean room wear, prepared separately, the value of S'_{E}/S'_{N} was 0.3147. In this case, when univariate discriminant analysis is performed, the discriminant value was 0.40, and the hazard rate was 0.09. That is, this indicates that if the value of S'_{E}/S'_{N} is greater than 0.40 the evaluation can be that the particle is a microorganism, with a hazard rate of 0.09.

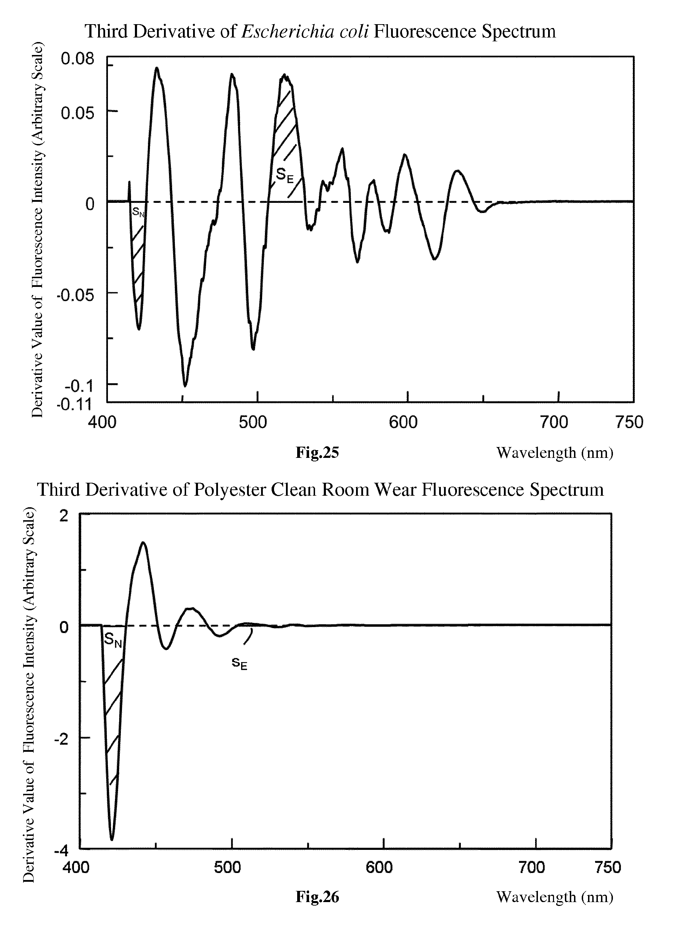

In the embodiment of a third derivative of the fluorescence peak for *E*. *coli,* shown in FIG. 25, and in the embodiment of a third derivative of the fluorescence spectrum of the polyester clean room wear shown in FIG. 26, the peak area S_{N} that appears in the vicinity of a wavelength of 420 nm is an area of a peak that is not significant, appearing due to the light of less than the 420 nm wavelength that is blocked by the long-pass filter when the derivative value 0 of the fluorescence intensity is used as the base. Moreover, in the embodiment of a third derivative of the fluorescence peak for *E*. *coli*, shown in FIG. 25, the peak area S_{E} that appears near the wavelength of 510 nm is an area of a peak that could derive from fluorescent light that is emitted from a microorganism, resulting from the riboflavin when the derivative value 0 of the fluorescence intensity is used as the base. If it is not clear whether a fluorescence spectrum is due to a microorganism or due to a non-microorganism particle, the peak area S_{E} that appears here the wavelength of 510 nm, in the embodiment of a third derivative of the fluorescence spectrum of the polyester clean room wear shown in FIG. 26 is an area of a peak that could derive from the fluorescence emitted from a microorganism as the result of the riboflavin when the derivative value 0 of the fluorescence intensity is used as the base.

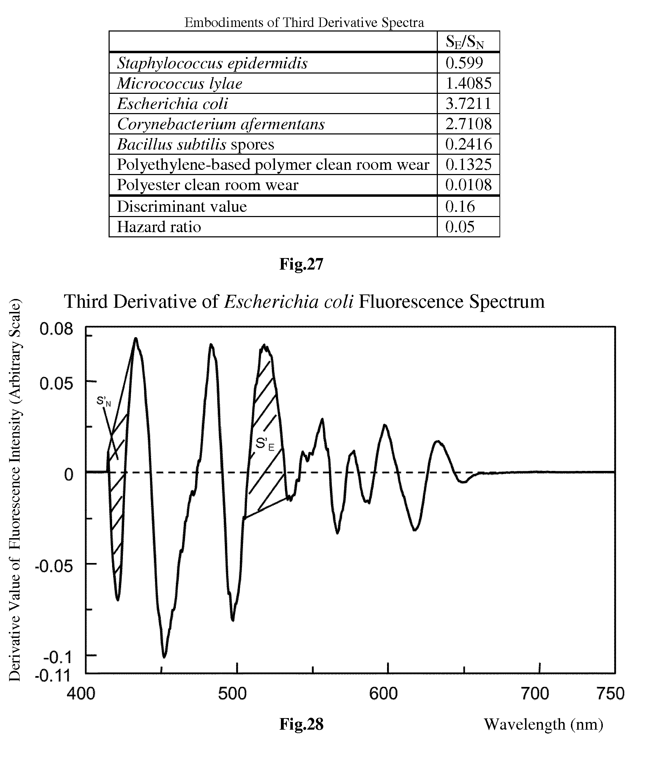

As shown in FIG. 27, the value for S_{E}/S_{N}, wherein the peak area S_{E} that appears near to the wavelength of 510 nm, which could derive from fluorescence emitted by a microorganism due to the riboflavin, in the embodiment of the third derivative of the fluorescence spectrum for *E. coli,* illustrated in FIG. 25, was divided by the area of the peak S_{N} that appears near to the wavelength of 420 nm, was 3.7211. In an embodiment of the third derivative of the fluorescence spectrum for *Staphylococcus epidermidis,* prepared separately, the value of S_{E}/S_{N} was 0.5990. In an embodiment of the third derivative of the fluorescence spectrum for *Micrococcus lylae,* prepared separately, the value of S_{E}/S_{N} was 1.4085. In an embodiment of the third derivative of the fluorescence spectrum for *Corynebacterium afermentans,* prepared separately, the value of S_{E}/S_{N} was 2.7108. In an embodiment of the third derivative of the fluorescence spectrum for *Bacillus subtilis* spores, prepared separately, the value of S_{E}/S_{N} was 0.2416.

In contrast, as illustrated in FIG. 27, the value for S_{E}/S_{N} in an embodiment of a third derivative of the fluorescence spectrum for the polyester clean room wear illustrated in FIG. 26 was 0.0108. In an embodiment of the third derivative of the fluorescence spectrum for the polyethylene-based polymer clean room wear, prepared separately, the value of S_{E}/S_{N} was 0.1325. In this case, when univariate discriminant analysis is performed, the discriminant value was 0.16, and the hazard rate was 0.05. That is, this indicates that if the value of S_{E}/S_{N} is greater than 0.16 the evaluation can be that the particle is a microorganism, with a hazard rate of 0.05.

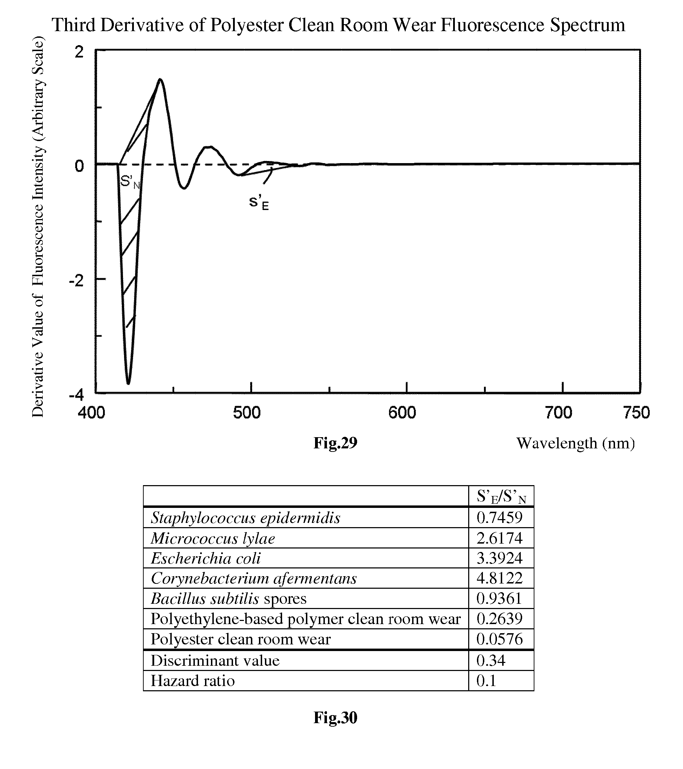

Next, in the embodiment of a third derivative of the fluorescence peak for *E. coli,* shown in FIG. 28, and in the embodiment of a third derivative of the fluorescence spectrum of the polyester clean room wear shown in FIG. 29, the peak area S'_{N} that appears in the vicinity of a wavelength of 420 nm is an area of a peak that is not significant, appearing due to the light of less than the 420 nm wavelength that is blocked by the long-pass filter when a line segment connecting the bottom edges of two peaks is used as the base. Moreover, in the embodiment of a third derivative of the fluorescence peak for *E. coli,* shown in FIG. 28, the peak area S'_{E} that appears near the wavelength of 510 nm is an area of a peak that could derive from fluorescent light that is emitted from a microorganism, resulting from the riboflavin when a line segment connecting the bottom edges of two peaks is used as the base. If it is not clear whether a fluorescence spectrum is due to a microorganism or due to a non-microorganism particle, the peak area S'_{E} that appears here the wavelength of 510 nm, in the embodiment of a third derivative of the fluorescence spectrum of the polyester clean room wear shown in FIG. 29 is an area of a peak that could derive from the fluorescence emitted from a microorganism as the result of the riboflavin when a line segment connecting the bottom edges of two peaks is used as the base.

As shown in FIG. 30, the value for S'_{E}/S'_{N}, wherein the peak area S'_{E} that appears near to the wavelength of 510 nm, which could derive from fluorescence emitted by a microorganism due to the riboflavin, in the embodiment of the third derivative of the fluorescence spectrum for *E. coli,* illustrated in FIG. 28, was divided by the area of the peak S'_{N} that appears near to the wavelength of 420 nm, was 3.3924. In an embodiment of the third derivative of the fluorescence spectrum for *Staphylococcus epidermidis,* prepared separately, the value of S'_{E}/S'_{N} was 0.7459. In an embodiment of the third derivative of the fluorescence spectrum for *Micrococcus lylae,* prepared separately, the value of S'_{E}/S'_{N} was 2.6174. In an embodiment of the third derivative of the fluorescence spectrum for *Corynebacterium afermentans,* prepared separately, the value of S'_{E}/S'_{N} was 4.8122. In an embodiment of the third derivative of the fluorescence spectrum for *Bacillus subtilis* spores, prepared separately, the value of S'_{E}/S'_{N} was 0.9361.

In contrast, as illustrated in FIG. 30, the value for S'_{E}/S'_{N} in an embodiment of a third derivative of the fluorescence spectrum for the polyester clean room wear illustrated in FIG. 29 was 0.0576. In an embodiment of the third derivative of the fluorescence spectrum for the polyethylene-based polymer clean room wear, prepared separately, the value of S'_{E}/S'_{N} was 0.2639. In this case, when univariate discriminant analysis is performed, the discriminant value was 0.34, and the hazard rate was 0.10. That is, this indicates that if the value of S'_{E}/S'_{N} is greater than 0.34 the evaluation can be that the particle is a microorganism, with a hazard rate of 0.10.

Conventionally, there has been a method for discriminating between microorganism particles and non-microorganism particles through performing multivariate analysis on the fluorescence spectrum. However, because multivariate analysis includes complex processes, the time required for calculation is long, and there is a tendency for the software to be burdensome. In contrast, in the microorganism detecting device 1 according to the present embodiment, it is possible to discriminate between microorganisms and non-microorganism particles more easily than with multivariate analysis.

### (Embodiment)

The embodiments explained for the forms above were obtained through the following methods.

### (Obtaining the Microorganisms)

The microorganisms obtained were *Escherichia coli* (abbreviated *"E. coli,"* ATCC 13706), *Staphylococcus epidermidis* (abbreviated *"S. epidermidis,* " ATCC 12228), *Bacillus atrophaeus* (abbreviated "*B. atrophaeus*," ATCC 9372), *Micrococcus lylae* (abbreviated *"M. lylae,"* ATCC 27566), and *Corynebacterium afermentans* (abbreviated *"C. afermentans,* "ATCC 51403. Note that ATCC is an abbreviation for the "American Type Culture Collection." The *E*. *coli* is a Gram-negative bacterium. *Staphylococcus epidermidis*, *Bacillus atrophaeus*, *Micrococcus lylae,* and *Corynebacterium afermentans* are Gram-positive bacteria.

### (Preparing the Microorganism Samples)

The *E*. *coli, S. epidermidis,* and *M. lylae* that were stocked at -80°C were inoculated into 150 mL each of a tryptic soy broth (Becton, Dickinson and Company, Ref: 211825) in a respective 300 mL Erlenmeyer flask, and left overnight at 32°C until a stationary phase was reached, to be cultured aerobically. Moreover, the *C*. *afermentans* was cultured in an R medium (10g peptone, 5g yeast extract, 5g malt extract, 5g casamino acid, 2g beef extract, 2g glycerin, 50 mg Tween 80,1g MgSO₄ - 7H₂O, and 1L distilled water, pH7.2) as a liquid culture medium.

Next, a centrifuge (Kubota Trading Company: 2410) was used to centrifuge each of the liquid media for 10 minutes at 2100 g to collect the bacteria, and then, after removing and discarding the supernatant medium, the microorganisms were resuspended in PBS. Moreover, this was centrifuged under the same conditions and the supernatant medium was removed to perform rinsing. This same rinsing was repeated a total of three times. For the *B. atrophaeus*, a commercially available spore solution (North American Science Associates, Inc.: SUN-07) was centrifuged under the same conditions to obtain the cells.

### (Measuring the Fluorescence Spectra)

The fluorescence spectra were measured using a fluorescence spectrophotometer FP 8500 (by Jasco Corporation). A plate of bacterial cells obtained through centrifuging was recovered and coated, in an adequate quantity, onto a slide glass. The slide glass was placed in a device for securing it to a film measurement holder of the fluorescence spectrophotometer. In the 3-D spectra (the excitation emission matrix) measurements, a long-pass filter with a cut off of 420 nm on the fluorescent side was used. Moreover, when measuring the fluorescence spectrum with optical excitation at 405 nm, an additional bandpass filter that passes 410 through 420 nm was used, to suppress scattering through stray light.

For the clean room wear, the fabric was held between glass slides, and was secured in a film measurement holder and measured. For the polyethylene material, Tyvek IsoClean (DuPont), was used, and for the polyester material, an ultrastatic clean suit hood (Midori Anzen) was used.

The calculations, such as the spectra differentiation, were performed using the Spectrum Manager software that came with the fluorescence spectrophotometer. Prior to the differentiation calculations, noise reduction was performed through performing simple moving averages on the spectra that were obtained.

### (Univariate Discriminant Analysis)

The absolute values of the peak heights (the derivative values of the fluorescent intensities) were calculated by the Spectrum Manager software based on the second and third derivative spectra of the fluorescence spectra. Moreover, the absolute values of the derivative values of the fluorescent intensities that can be derived from fluorescence emitted by fluorescent substances included in microorganisms were divided by the absolute values of the derivative values of the magnitudes of the fluorescent intensities that are not significant in the derivative spectra. Thereafter, univariate discriminant analysis was performed using the Microsoft spreadsheet software (Excel), to produce the results explained in the embodiment.

Moreover, the absolute values of the peak areas were calculated by the Spectrum Manager software based on the second and third derivative spectra of the fluorescence spectra. Moreover, the peak areas can be derived from fluorescence emitted by fluorescent substances included in microorganisms were divided by the peak areas that are not significant in the derivative spectra. Thereafter, univariate discriminant analysis was performed using the Microsoft spreadsheet software (Excel), to produce the results explained in the embodiment.

### (Other Forms of Embodiment)

While there are descriptions of forms of embodiment as set forth above, the descriptions and drawings that form a portion of the disclosure are not to be understood to limit the present invention. A variety of alternate examples of embodiment and operating technologies should be obvious to those skilled in the art. For example, the microorganism detecting device 1, illustrated in FIG. 2, may further comprise a scattered light detecting device. The scattered light detecting device detects the light that is scattered by the particles that are included in the gas. The strength of the light that is scattered by a particle is correlated with the size of the particle. Consequently, it is possible to calculate the diameter of a particle that is included in the airflow by detecting the intensity of the scattered light that is detected by the scattered light detecting device. In this way, the present invention should be understood to include a variety of forms of embodiment, and the like, not set forth herein.

List of reference signs
- 1:: Microorganism Detecting Device
- 21:: Frame
- 22:: First Vacuum Device
- 23:: Nozzle
- 24:: Second Vacuum Device
- 25:: Light Source
- 26:: Laser Beam
- 27:: Fluorescence Detector
- 70:: Clean Room
- 71:: Duct
- 72:: Spraying Opening

- 81:: Manufacturing Line
- 82:: Manufacturing Line
- 300:: CPU
- 301:: Evaluating Portion

## Claims

1. A microorganism detecting device (1) comprising:
a light source (25) that illuminates a particle with an excitation beam;
a fluorescence detector (27) that detects produced by the particle;
an evaluating portion (301) adapted to evaluate that the particle is a microorganism if there is a plurality of peaks in the fluorescence spectrum, and adapted to evaluate that the particle is a non-microorganism if there is a single peak in the fluorescence spectrum.

2. The microorganism detecting device as set forth in Claim 1, wherein:
the evaluating portion (301) is adapted to calculate a derivative of the fluorescence spectrum.

3. The microorganism detecting device as set forth in Claim 1 or Claim 2, wherein:
the evaluating portion (301) is adapted to calculate a multiple derivative of the fluorescence spectrum.

4. The microorganism detecting device as set forth in Claim 2 or Claim 3, wherein:
the evaluating portion (301) is adapted to compare, to a discriminant value that is set in advance, an absolute value of a derivative value of a fluorescent intensity of a peak that could derive from fluorescence emitted from a microorganism in the derivative spectrum where the derivative of the fluorescence spectrum has been taken, and to evaluate a particle as a microorganism if an absolute value of the derivative value of the fluorescent intensity is larger than the discriminant value, and to evaluate the particle to be a non-microorganism particle if the absolute value of the derivative value of the fluorescent intensity is smaller than the discriminant value.

5. The microorganism detecting device as set forth in Claim 4, wherein:
the evaluating portion (301) is adapted to divide the absolute values of the derivative values of the fluorescent intensities that can be derived from microorganisms by the absolute values of the derivative values of magnitudes of the fluorescent intensities that are not significant in a derivative spectrum.

6. The microorganism detecting device as set forth in Claim 2 or Claim 3, wherein:
the evaluating portion (301) is adapted to compare, to a discriminant value that is set in advance, an area of a peak that could derive from fluorescence emitted from a microorganism in the derivative spectrum where the derivative of the fluorescence spectrum has been taken, and to evaluate a particle as a microorganism if the area of the peak is larger than the discriminant value, and to evaluate the particle to be a non-microorganism particle if the area of the peak is smaller than the discriminant valuewherein, in particular, :
the evaluating portion (301) is adapted to divide the area of a peak that can be derived from microorganisms by an area of a peak of the fluorescent intensities that are not significant in a derivative spectrum.

7. The microorganism detecting device as set forth in any one of Claim 1 through Claim 6, further comprising:
a flow path wherein a fluid that includes a particle flows at a location that is illuminated by the excitation beam.

8. The microorganism detecting device as set forth in any one of Claim 1 through Claim 7, wherein:
the excitation beam has a wavelength between 250 and 550 nm.

9. A microorganism detecting method, wherein:
a particle is illuminated with an excitation beam;
fluorescence produced by the particle is detected; and
a particle is evaluated to be a microorganism if there is a plurality of peaks in a fluorescence spectrum, and the particle is evaluated to be a non-microorganism particle there is a single peak in the fluorescence spectrum.

10. The microorganism detecting method as set forth in Claim 9, wherein:
in the evaluation, a derivative of the fluorescence spectrum is calculated.

11. The microorganism detecting method as set forth in Claim 9 or Claim 10, further wherein:
in the evaluation, a multiple derivative of the fluorescence spectrum is calculated.

12. The microorganism detecting method as set forth in Claim 10 or Claim 11, further wherein:
the absolute value of the derivative value of the fluorescent intensity of a peak that could derive from fluorescence emitted from a microorganism in the derivative spectrum where the derivative of the fluorescence spectrum has been taken is compared to a discriminant value that is set in advance, and a particle is evaluated to be a microorganism if an absolute value of the derivative value of the fluorescent intensity is larger than the discriminant value, and the particle is evaluated to be a non-microorganism particle if an absolute value of the derivative value of the fluorescent intensity is smaller than the discriminant value, wherein in particular
the absolute values of the derivative values of the fluorescent intensities that can be derived from microorganisms are divided by the absolute values of the derivative values of the magnitudes of the fluorescent intensities that are not significant in a derivative spectrum.

13. The microorganism detecting method as set forth in Claim 10 or Claim 11, further wherein:
an area of a peak that could derive from fluorescence emitted from a microorganism in the derivative spectrum where the derivative of the fluorescence spectrum has been taken is compared to a discriminant value that is set in advance, and a particle is evaluated to be a microorganism if the area of the peak is larger than the discriminant value, and the particle is evaluated to be a non-microorganism particle if the area of the peak is smaller than the discriminant value, wherein, in particular,
the area of a peak that can be derived from microorganisms is divided by the area of a peak of the fluorescent intensities that are not significant in a derivative spectrum.

14. The microorganism detecting method as set forth in any one of Claim 9 through Claim 13, further including:
a fluid that includes a particle flowing at a location that is illuminated by the excitation beam.

15. The microorganism detecting method as set forth in any one of Claim 9 through Claim 14, wherein:
the excitation beam has a wavelength between 250 and 550 nm.
